# EUROPÄISCHE PATENTSCHRIFT

(11) **EP 1 101 447 B1**
(45) Veröffentlichungstag und Bekanntmachung des Hinweises auf die Patenterteilung: **27.04.2005**
(21) Anmeldenummer: 00115179.4
(22) Anmeldetag: 13.07.2000
(51) Int. Cl.: A61B 17/34

(54) **Dichtung für ein medizinisches Instrument**
Seal for a medical instrument
Joint d'étanchéité pour un instrument médical

(30) Priorität: 15.11.1999 DE 19955071
(43) Veröffentlichungstag der Anmeldung: 23.05.2001
(73) Patentinhaber: Richard Wolf GmbH, 75438 Knittlingen (DE)
(72) Erfinder: Kunert, Benno, 75177 Pforzheim (DE); Wagner, Carl-Sebastian, 75015 Bretten (DE)
(74) Vertreter: Wilcken, Thomas, Dipl.-Ing.

(56) Entgegenhaltungen:
- EP-A- 0 893 099
- WO-A-94/17844
- US-A- 5 628 732
- US-A- 5 741 298

## Beschreibung

Die Erfindung betrifft eine Dichtung für ein medizinisches Instrument, bestehend aus einem Dichtungselement mit einer zentrisch in einer elastischen Membran vorgesehenen, elastisch aufweitbaren Öffnung zum dichten Einführen und Durchführen eines Hilfsinstruments in und durch einen Kanal des Instruments wo das Dichtungselement mit einem randseitig umlaufenden, die Membran haltenden Träger an einer durch einen proximal am Instrument radial abstehenden Flansch gebildeten Aufnahme radial versetzbar geführt werden kann, und ein Kragen des Dichtungselementes distalseitig unter den Flansch greifen kann und an diesem anliegen kann.

Solche Dichtungen werden bei medizinischen Instrumenten, wie Trokarhülsen, Kanülen, Kathetern und dergleichen, verwendet, um Hilfsinstrumente abgedichtet durch den Instrumentenkanal bewegen zu können. Damit bei Anwendung von Hilfsinstrumenten unterschiedlicher Durchmesser während einer Untersuchung oder Operation die jeweilige Dichtung nicht gegen eine andere mit kleinerer oder größerer Membranöffnung ausgewechselt werden muss, besteht die Membran zumindest im Bereich ihrer Öffnung aus hochelastischem und hochreißfestem Material auf Silikonbasis, Latexbasis oder auf der Basis thermoplastischer Elastomere.

So kann erreicht werden, dass die Membranöffnung um ein Mehrfaches ihres Ausgangsdurchmessers aufgeweitet werden kann, wenn Hilfsinstrumente mit entsprechend großen Durchmessern durch das Dichtungselement gesteckt und weiter durch den Instrumentenkanal mit ihrem distalen Ende bis in eine Körperhöhle geschoben werden sollen. Im Übrigen ist der Ausgangsdurchmesser der unbelasteten Membranöffnung kleiner als der des jeweiligen Hilfsinstruments mit kleinstem Durchmesser, um auch ein solches Instrument bei nur relativ gering aufgeweiteter Membranöffnung ausreichend abgedichtet in und durch den Instrumentenkanal bewegen zu können.

Man kann davon ausgehen, dass eine ausreichende Dichtungsfunktion gewährleistet sein wird, wenn die Membranöffnung, das medizinische Instrument und das über die Dichtung eingeführte Hilfsinstrument aufgleicher Achse liegen, weil dann die Membran im Bereich ihrer Öffnung gleichmäßig aufgeweitet ist und über den Umfang des Hilfsinstruments gesehen überall mit gleichem Druck dichtend gegen das Hilfsinstrument anliegen wird.

Dichtungsprobleme treten aber auf, wenn mit dem Hilfsinstrument etwa bei einem chirurgischen Eingriff so manipuliert wird, dass das Hilfsinstrument z. B. in einer Trokarhülse relativ zu deren Längsachse in eine Schrägstellung gebracht werden muss, um mit dem distalen Ende des Hilfsinstruments das Operationsfeld erreichen zu können, da die Trokarhülse hierbei im Wesentlichen unbeweglich in einem Einstich in der Bauchdecke des Patienten steckt.

In solchen auch bei anderen Instrumenten als Trokarhülsen gegebenen Fällen wird eine fest am Umfang eingespannte bzw. gehaltene Membran im Öffnungsbereich ungleichmäßig belastet und verformt, so dass sie streckenweise nur noch mit geringer und nicht mehr ausreichenden Dichtkraft am Umfang des Hilfsinstruments anliegen oder sogar außer Kontakt mit dem Hilfsinstrument gelangen wird, so dass beispielsweise in eine Körperhöhle eingeführtes Gas ungewollt über das Instrument nach außen abströmen wird.

Dieses Problem gibt es nicht, wenn die Membran bzw. der sie am Randbereich haltende Träger bei Manipulationen mit dem Hilfsinstrument radial aufgrund der auf die Membran einwirkenden Kräfte versetzbar ist und das Dichtungselement somit seitlich ausweichen kann, ohne dass es dabei zu wesentlichen ungleichmäßigen Verformungen der Membran kommen kann. Bei bekannten Dichtungen dieser Art (WO94/17844, US 5,628,732) wird die elastische Membran von einem starren Träger gehalten, der radial in einer zur Instrumentenlängsachse offenen, proximal im Instrumentengehäuse vorgesehenen Ringnut als Aufnahme für das Dichtungselement verschoben werden kann, so dass der Träger mit der Membran, also das Dichtungselement insgesamt, durch seitlichen Versatz einer außermittig gerichteten Stellungsänderung des Hilfsinstruments im Dichtungsbereich folgen kann und es deshalb nicht zu problematischen Verformungen der Membran mit der Folge von Leckagen kommen wird.

Starre Membranträger lassen sich in der Ringnut zwar gut radial führen, sie können aber selbst nichts Wesentliches zu einer Dichtung zwischen dem Instrumentengehäuse und dem Dichtungselement beitragen. Deshalb ist ein weiteres Dichtungsteil z. B. in Form eines ringförmigen Faltenbalgs erforderlich, über den der Träger mit der ihn aufnehmenden Ringnut dicht verbunden ist. Das mehrteilig aus Membran, Träger und Faltenbalg bestehende Dichtungselement ist allerdings relativ aufwendig und teuer.

Ein weiterer wesentlicher Nachteil solcher Dichtungen ist darin zu sehen, dass zu ihrer Montage und Demontage vorab deckelartige Teile des Instrumentengehäuses abgebaut werden müssen, um den Membranträger mit zusätzlichem Dichtungsteil in die Aufnahme bzw. Ringnut einsetzen bzw. umgekehrt wieder entnehmen zu können, wobei auch noch beim Montagevorgang auf eine exakte Ausrichtung der einzubauenden Teile auf das Instrumentengehäuse zu achten ist.

Die Aufgabe der Erfindung besteht in der Beseitigung der aufgezeigten Nachteile und somit in der Schaffung einer in ihrem Aufbau einfachen und kostengünstig zu fertigenden Dichtung, die sich besonders schnell montieren und demontieren lässt, ohne dass dabei an dem mit der Dichtung auszustattenden Instrument manipuliert werden muss.

Zur Lösung dieser Aufgabe wird die eingangs erwähnte Dichtung erfindungsgemäß so ausgebildet, dass das Dichtungselement eine elastische Dichtlippe aufweist, die mit dem Druck im Instrumentenkanal beaufschlagt werden kann und die proximalseitig auf dem Flansch anliegen kann.

Der als Dichtungsaufnahme dienende und das proximale Ende des Instruments bildende Flansch ist bei einigen medizinischen Geräten, wie etwa bei Trokarhülsen, meist schon vorhanden oder kann sonst einfach in einem Arbeitsgang mit dem Instrumentengehäuse hergestellt werden. Das Dichtungselement wird bei seiner Montage von Hand am Flansch angesetzt und dann durch axial gerichteten Druck weiter distalwärts bewegt, bis der sich dabei elastisch verformende Kragen schließlich hinter den Flansch schnappt und die Dichtlippe proximal auf dem Flansch zu liegen kommt. Auch die Demontage des Dichtungselements kann mit einem Handgriff erfolgen, indem es einfach vom Flansch abgezogen wird.

Das Dichtungselement lässt sich kostengünstig als einstückiges Spritzgussteil aus elastischem Material herstellen und hat im Wesentlichen die Form einer Kappe, die den Rand des Flansches übergreift.

Die Membran ist in bekannter Weise trichterförmig ausgebildet und verjüngt sich distalwärts in Richtung auf ihre Öffnung, so dass an der Membran angesetzte Hilfsinstrumente beim Einführen in das medizinische Instrument zwangsläufig mit ihrem distalen Ende zur Membranöffnung hin geführt werden.

Außerdem ist die Membran im Bereich ihrer Trichterform zumindest proximalseitig mit einer Beschichtung aus einem Material mit niedrigem Reibungskoeffizienten versehen, damit das betreffende Hilfsinstrument leicht gleitend in der einen oder anderen Richtung durch die Membranöffnung bewegt werden kann. Schließlich ist die Membran, ausgehend von ihrer Öffnung bis zum Träger, in der Wandstärke dicker werdend ausgebildet, wodurch die Membran vor allem am Randbereich gegen Beschädigungen durch ein einzuführendes Hilfsinstrument geschützt ist und nach Entnahme eines Hilfsinstruments selbsttätig wieder die vorgeprägte Trichterform einnehmen wird.

Nachfolgend wird ein in der Zeichnung schematisch dargestelltes Ausführungsbeispiel der Erfindung beschrieben. In der Zeichnung zeigt:
- Fig. 1: einen Längsschnitt durch ein medizinisches Instrument mit einer erfindungsgemäßen Dichtung und
- Fig. 2 und 3: das gleiche Instrument wie in Fig. 1 mit durch die Dichtung und das Instrument geschobenen Hilfsinstrumenten unterschiedlicher Durchmesser.

Beim dargestellten Instrument handelt es sich um eine Trokarhülse 1 mit einem Gehäuse 2 und einem Schaft 3, die einen Kanal 4 zum Einführen eines Hilfsinstruments in eine Körperhöhle bilden. Unmittelbar am proximalen Ende hat das Instrument 1 einen radial nach außen abstehenden Flansch 5, der als Aufnahme für ein Dichtungselement 6 dient, das mit einem randseitig umlaufenden, eine Membran 7 einfassenden und haltenden Träger 8 am Flansch 5 radial versetzbar geführt ist.

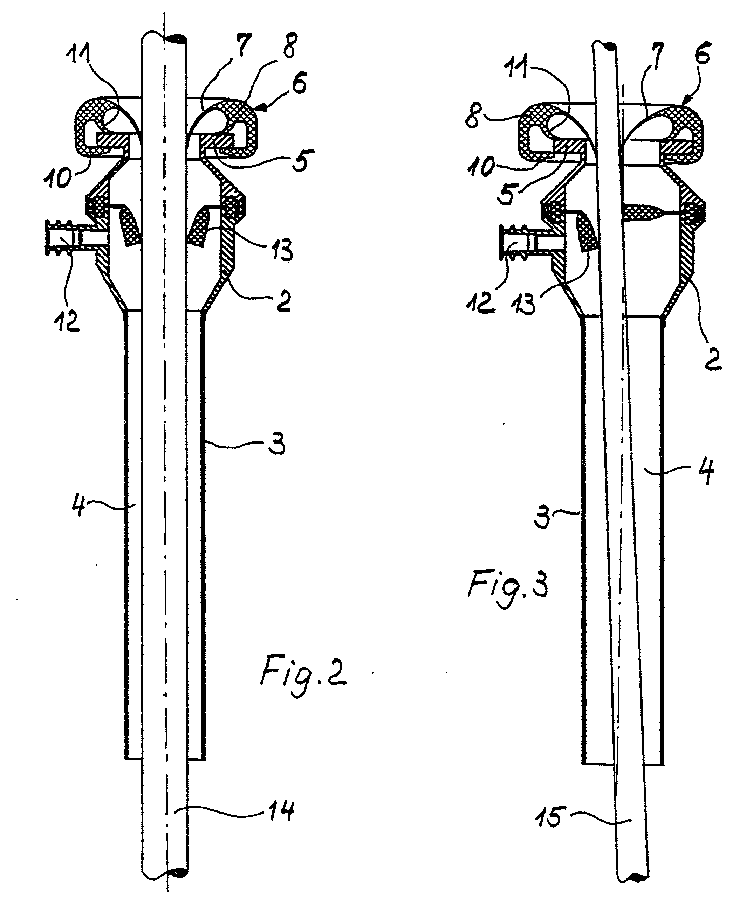

Zentrisch in der Membran 7 ist eine Öffnung 9 vorgesehen, durch die ein Hilfsinstrument abgedichtet in und durch den Instrumentenkanal 4 geschoben werden kann, wie auch noch später im Zusammenhang mit den Fig. 2 und 3 weiter beschrieben wird.

Der Träger 8 besteht zwar wie die Membran 7 und das Dichtungselement 6 insgesamt aus einem hochelastischem Material, er ist aber wegen seiner vergleichsweise großen Wandstärke relativ formstabil und übergreift beim montierten Dichtungselement 6 den Flansch 5 so, dass ein Kragen 10 distalseitig unten am Flansch 5 anliegt und eine vom Träger 8 ausgehende Dichtlippe 11 elastisch verformt oben bzw. proximalseitig gegen den Flansch 5 gedrückt ist. Aufgrund ihrer dargestellten Form und Position wird die Dichtlippe 11 mit einem im Instrumentenkanal 4 herrschenden Überdruck eines in die Körperhöhle insufflierten Gases beaufschlagt, das über einen Anschluss 12 in das Instrument 1 und weiter distalwärts durch den Schaft 3 in die Körperhöhle eingeführt wurde.

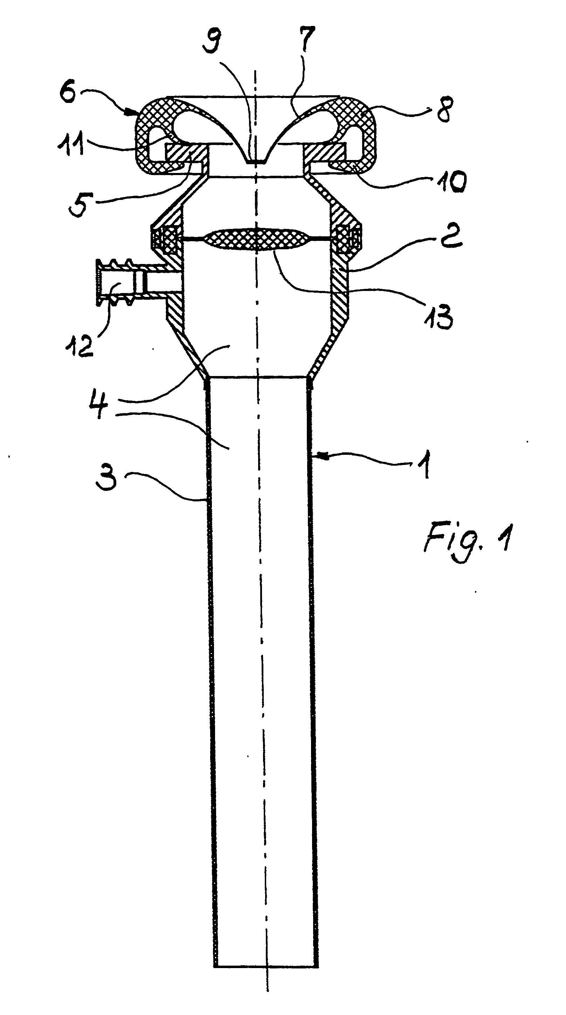

Damit das Gas nicht über das Instrument entweichen kann, wenn die Öffnung 9 gemäß Fig. 1 nicht durch ein Hilfsinstrument verschlossen ist, wird der Instrumentenkanal 4 im Gehäuse 2 üblicherweise mit einem Ventil 13 abgesperrt, das beispielsweise als Schlitzmembranventil ausgebildet ist und entsprechend den Fig. 2 und 3 von einem Hilfsinstrument passiert werden kann.

Wenn nach Fig. 2 ein Hilfsinstrument 14 mit relativ großem Durchmesser durch das Dichtungselement 6 distalwärts in das Instrument 1 eingeführt wird, wird das freie Lumen des Schaftkanals 4 im Wesentlichen vom Hilfsinstrument 14 eingenommen, so dass wenig Spielraum für seitwärtige Bewegungen bzw. Kippbewegungen des Hilfsinstruments in der Trokarhülse 1 gegeben ist und das Dichtungselement 6 in diesem Fall seine auch in Fig. 1 gezeigte Position beibehalten wird, und zwar bei stark aufgeweiteter Öffnung 9.

Dagegen sind Manipulationsbewegungen des Hilfsinstruments eher möglich, wenn dieses einen vergleichsweise kleinen Durchmesser hat. Die Fig. 3 zeigt ein solches Hilfsinstrument 15 in einer im Instrumentenkanal 4 gekippten Stellung. Eine zu dieser Stellung führende Kippbewegung wird der Fig. 3 entsprechend aufgrund der auf die Membran 7 einwirkenden Kräfte einen seitlichen Versatz des Dichtungselements 6 zur Folge haben, wobei der Kragen 10 im Zusammenwirken mit dem Flansch 5 eine Führungsfunktion übernimmt und die ringförmige Dichtlippe 11 auf dem Flansch 5 seitwärts in die Position gemäß Fig. 3 gleitet, so dass das Dichtungselement 6 insgesamt eine der seitlichen Bewegung des Hilfsinstruments 15 folgende Ausgleichsbewegung durchführt und es hierbei nicht dazu kommen kann, dass die Membran 7 im Bereich ihrer Anlage gegen den Umfang des Hilfsinstruments 15 so stark verformt werden könnte, dass die Membran über den Umfang des Hilfsinstruments gesehen ungleichmäßig gegen das Hilfsinstrument drückt oder sogar unter Bildung einer Leckage stellenweise außer Kontakt mit dem Hilfsinstrument kommt.

Es ist zu erwarten und auch verständlich, dass bei im Vergleich zu Fig. 3 weniger extremen Kippbewegungen beim Manipulieren mit dem Hilfsinstrument 15 nicht immer das Dichtungselement 6 insgesamt seitlich versetzt und verschoben wird, sondern nur der obere Teilbereich des Trägers 8 mit der Dichtlippe 11, während der Kragen 10 in seiner Ausgangsposition gemäß Fig. 1 verbleiben und erst bei größer werdenden Kippstellungen des Hilfsinstruments im Instrumentenkanal 4 ebenfalls die seitliche Ausgleichsbewegung mitmacht.

Wie schon erwähnt wurde, wird durch die Trichterform der Membran 7 das Einführen des Hilfsinstruments in das Dichtungselement erleichtert. Außerdem bietet diese Trichterform den Vorteil, dass sich die Membran 7 im Bereich der Öffnung 9 großflächig und eng am Hilfsinstrumentenumfang anlegen wird, wenn die Membran in diesem Bereich entsprechend dünnwandig und somit hochelastisch ist. Zum Träger 8 hin sollte die Membran dagegen den Darstellungen entsprechend allmählich dicker werdend ausgebildet sein, wodurch die Membran ausreichend formstabil ist und auch nach Herausziehen eines Hilfsinstruments wieder die in Fig. 1 gezeigte Form einnehmen wird.

## Patentansprüche

1. Dichtung für ein medizinisches Instrument (1), bestehend aus einem Dichtungselement (6) mit einer zentrisch in einer elastischen Membran (7) vorgesehenen, elastisch aufweitbaren Öffnung (9) zum dichten Durchführen und Einführen eines Hilfsinstruments (14, 15) in und durch einen Kanal (4) des Instruments, wo das Dichtungselement (6) mit einem randseitig umlaufenden, die Membran (7) haltenden Träger (8) an einer durch einen proximal am Instrument (1) radial abstehenden Flansch (5) gebildeten Aufnahme (5) radial versetzbar geführt werden kann, und ein Kragen (10) des Dichtungselementes (6) distalseitig unter den Flansch (5) greifen kann und an diesem anliegen kann,
**dadurch gekennzeichnet, dass** das Dichtungselement (6) eine elastische Dichtlippe (11) aufweist, die mit dem Druck im Instrumentenkanal (4) beaufschlagt werden kann und die proximalseitig auf dem Flansch (5) anliegen kann.

2. Dichtung nach Anspruch 1, **dadurch gekennzeichnet, dass** das Dichtungselement (6) als auf das Instrument (1) setzbare Kappe ausgebildet ist, die den Rand des Flansches (5) übergreifen kann.

3. Dichtung nach einem der Ansprüche 1 und 2, **dadurch gekennzeichnet, dass** das Dichtungselement (6) ein einstückiges Spritzgussteil aus elastischem Material ist.

4. Dichtung nach einem der Ansprüche 1 bis 3, **dadurch gekennzeichnet, dass** die Membran (7) trichterförmig ist und sich distalwärts in Richtung auf ihre Öffnung (9) verjüngt.

5. Dichtung nach einem der Ansprüche 1 bis 4, **dadurch gekennzeichnet, dass** die Membran (7) mit einer Schicht aus einem Material mit niedrigem Reibungskoeffizienten versehen ist.

6. Dichtung nach einem der Ansprüche 1 bis 5, **dadurch gekennzeichnet, dass** die Membran (7), ausgehend von ihrer Öffnung (9) bis zum Träger (8), dicker werdend ausgebildet ist.

## Claims

1. A seal for a medical instrument (1), consisting of a sealing element (6) with an elastically widenable opening (9) provided centrically in an elastic membrane (7) for the sealed leading-through and introduction of an auxiliary instrument (15, 15) in and through a channel (4) of the instrument where the sealing element (6) with a carrier (8) which is peripheral on the edge and which holds the membrane (7), may be guided in a radially displaceable manner on a receiver (5) which is formed by a flange (5) projecting radially and proximally on the instrument (1), and a collar (10) of the sealing element (6), on the distal side may engage below the flange (5) and bear on this flange,
**characterised in that** the sealing element (6) comprises an elastic sealing lip (11) which may be subjected to the pressure in the instrument channel (4) and which may bear on the flange (5) on the proximal side.

2. A seal according to claim 1, **characterised in that** the sealing element (6) is designed as a cap which may be placed onto the instrument (1) and which may engage over the edge of the flange (5).

3. A seal according to one of the claims 1 and 2, **characterised in that** the sealing element (6) is a one-piece injection moulded part of elastic material.

4. A seal according to one of the claims 1 to 3, **characterised in that** the membrane (7) is funnel-like and tapers distally in the direction of its opening (9).

5. A seal according to one of the claims 1 to 4, **characterised in that** the membrane (7) is provided with a layer of a material with a low friction coefficient.

6. A seal according to one of the claims 1 to 5, **characterised in that** the membrane (7) is designed becoming thicker, proceeding from its opening (9) up to the carrier (8).

## Revendications

1. Joint d'étanchéité destiné à un instrument médical (1), constitué d'un élément d'étanchéité (6) muni d'une ouverture (9) élastique extensible, située au centre d'une membrane élastique (7), permettant l'introduction et le passage en toute étanchéité d'un instrument auxiliaire (14, 15) dans et à travers un canal (4) de l'instrument, dans lequel l'élément d'étanchéité (6) peut, au moyen d'un support (8), supportant la membrane (7) et ayant un bord circulaire, être guidé selon un mouvement radial sur un récepteur (5) formé par une bride (5) faisant saillie radialement proximale à l'instrument (1), et dans lequel une collerette (10) de l'élément d'étanchéité (6) peut venir en contact sous la surface distale de la bride (5) et s'y ajuster étroitement, **caractérisé en ce que** l'élément d'étanchéité (6) présente une lèvre d'étanchéité (11) élastique, qui peut, sous l'effet de la pression à l'intérieur du canal (4) de l'instrument, être repoussée pour s'ajuster étroitement sur le côté proximal de la bride (5).

2. Joint d'étanchéité selon la revendication 1, **caractérisé en ce que** l'élément d'étanchéité (6) est construit sous la forme d'une coiffe sur l'instrument (1), laquelle peut enserrer le bord de la bride (5).

3. Joint d'étanchéité selon l'une quelconque des revendications 1 ou 2, **caractérisé en ce que** l'élément d'étanchéité (6) est obtenu par moulage par injection en une seule pièce à partir d'un matériau élastique.

4. Joint d'étanchéité selon l'une quelconque des revendications 1 à 3, **caractérisé en ce que** la membrane (7) est en forme d'entonnoir et se rétrécit en direction distale de son ouverture (9).

5. Joint d'étanchéité selon l'une quelconque des revendications 1 à 4, **caractérisé en ce que** la membrane (7) est pourvue d'une surface en matériau à faible coefficient de frottement.

6. Joint d'étanchéité selon l'une quelconque des revendications 1 à 5, **caractérisé en ce que** la membrane (7) présente une épaisseur croissante de son ouverture (9) jusqu'au support (8).
